# EUROPEAN PATENT APPLICATION

(11) **EP 3 915 497 A2**
(43) Date of publication of application: **01.12.2021**
(21) Application number: 21176079.8
(22) Date of filing: 26.05.2021
(51) Int. Cl.: A61B 17/34

(54) **SURGICAL ACCESS DEVICE WITH AN ANCHOR AND A REMOVABLE RETAINER**

(30) Priority: 27.05.2020 US 202016884706
(71) Applicant: Covidien LP, Mansfield, MA 02048 (US)
(72) Inventor: GEORGE, Sabastian Koduthully, 562125 BANGALORE (IN); BUYDA, Oksana, East Haven, CT 06513 (US)
(74) Representative: Maschio, Antonio

(57) **Abstract**

A surgical access device includes an instrument valve housing including a cannula extending distally from the instrument valve housing. The cannula defines a longitudinal axis. An anchor is supported at a distal end portion of the cannula. A retainer is configured to be removably engaged with cannula. The retainer includes a first member defining a semi-circular shape. The first member defines a first arm having a first lateral surface, and a second arm having a second lateral surface. A first magnet is positioned in the first arm and a second magnet is positioned in the second arm. A second member defines a semi-circular shape. The second member defines a third arm having a third lateral surface, and a fourth arm having a fourth lateral surface. A third magnet is positioned in the third arm and a fourth magnet is positioned in the fourth arm.

## Description

### FIELD

The present disclosure relates to a surgical access device. More particularly, the present disclosure relates to a surgical access device having an anchor and a removable retainer to help maintain a position of the surgical access device relative to a patient during a surgical procedure.

### BACKGROUND

In minimally invasive surgical procedures, including endoscopic and laparoscopic surgeries, a surgical access device permits the introduction of a variety of surgical instruments into a body cavity or opening. A surgical access device (e.g., a cannula or an access port) is introduced through an opening in tissue (e.g., a naturally occurring orifice or an incision) to provide access to an underlying surgical site in the body. The opening is typically made using an obturator having a blunt or sharp tip that may be inserted through a passageway of the surgical access device. For example, a cannula has a tube of rigid material with a thin wall construction, through which an obturator may be passed. The obturator is utilized to penetrate a body wall, such as an abdominal wall, or to introduce the surgical access device through the body wall, and is then removed to permit introduction of surgical instruments through the surgical access device to perform the surgical procedure.

Minimally invasive surgical procedures, including both endoscopic and laparoscopic procedures, permit surgery to be performed on organs, tissues, and vessels far removed from an opening within the tissue. In laparoscopic procedures, the abdominal cavity is insufflated with an insufflation gas, e.g., CO₂, to create a pneumoperitoneum thereby providing access to the underlying organs. A laparoscopic instrument is introduced through a cannula accessing the abdominal cavity to perform one or more surgical tasks. The cannula may incorporate a seal to establish a substantially fluid tight seal about the laparoscopic instrument to preserve the integrity of the pneumoperitoneum. The cannula, which is subjected to the pressurized environment, e.g., the pneumoperitoneum, may include an anchor mechanism to prevent the cannula from backing out of the opening in the abdominal wall, for example, during manipulation of the laparoscopic instrument within the cannula or withdrawal of the laparoscopic instrument therefrom.

Accordingly, it may be helpful to provide a retainer to help maintain the longitudinal position of the surgical access device with respect to the patient.

### SUMMARY

The present disclosure relates to a surgical access device including an instrument valve housing including a cannula extending distally from the instrument valve housing. The cannula defines a longitudinal axis. An anchor is supported at a distal end portion of the cannula. A retainer is configured to be removably engaged with the cannula. The retainer includes a first member defining a semi-circular shape. The first member includes a first arm having a first lateral surface and a second arm having a second lateral surface. A first magnet is positioned in the first arm and a second magnet is positioned in the second arm. A second member defines a semi-circular shape. The second member includes a third arm having a third lateral surface and a fourth arm having a fourth lateral surface. A third magnet is positioned in the third arm and a fourth magnet is positioned in the fourth arm. The first lateral surface of the first member is configured to contact the third lateral surface of the second member. The second lateral surface of the first member is configured to contact the fourth lateral surface of the second member. A first attractive force between the first magnet and the third magnet, and a second attractive force between the second magnet and the fourth magnet releaseably couples the first member to the second member such that the retainer frictionally engages with the cannula.

In aspects, the first member includes a first inner surface defining first ridges. The second member includes a second inner surface defining second ridges. The first and second ridges are configured to frictionally engage the cannula.

In aspects, a first outer surface of the first magnet defines a first continuously planar surface with the first lateral surface of the first member. A second outer surface of the second magnet forms a second continuously planar surface with the second lateral surface of the first member. A third outer surface of the third magnet forms a third continuously planar surface with the third lateral surface of the second member. A fourth outer surface of the fourth magnet forms a fourth continuously planar surface with the fourth lateral surface of the second member.

In aspects, the anchor includes a balloon anchor. A port supported on the instrument valve housing is configured to provide inflation fluid to the balloon anchor.

In aspects, the retainer is formed of rubber, plastic or a polymer.

The present disclosure also relates to a surgical access device including an instrument valve housing including a cannula extending distally from the instrument valve housing. The cannula defines a longitudinal axis. An anchor is supported at a distal end portion of the cannula. A retainer is configured to be removably frictionally engaged with the cannula. The retainer includes an annular body defining a first body portion and a second body portion. A serrated edge is formed in the annular body between the first body portion and the second body portion. The serrated edge is configured to facilitate cutting of the annular body to separate the first body portion from the second body portion to remove the retainer from the cannula.

In aspects, a second serrated edge is formed in the first body portion and a third serrated edge is formed in the second body portion.

Other features of the disclosure will be appreciated from the following description.

### DESCRIPTION OF THE DRAWINGS

The accompanying drawings, which are incorporated in and constitute a part of this specification, illustrate aspects and features of the disclosure and, together with the detailed description below, serve to further explain the disclosure, in which:
FIG. 1 is a perspective view of a surgical access device including a retainer according to an aspect of the present disclosure;
FIG. 2 is a perspective view of the retainer of FIG. 1;
FIG. 3 is an exploded view of the retainer separated from a cannula of the surgical access device of FIG. 1;
FIG. 4 is perspective view of the retainer of FIG. 2 in a separated configuration;
FIG. 5 is a cross-sectional view taken along section line 5-5 of FIG. 1;
FIG. 6A is a perspective view of a first member of the retainer of FIG. 1 with magnets shown in phantom;
FIG. 6B is a perspective view of a second member of the retainer of FIG. 1 with magnets shown in phantom;
FIG. 6C is a perspective view of the first member of FIG. 6A secured to the second member of FIG. 6B with magnets shown in phantom;
FIG. 7 is a cross-sectional view taken along section line 7-7 of FIG. 5;
FIG. 8 is a perspective view of a retainer according to another aspect of the present disclosure; and
FIG. 9 is a side view of the surgical access device of FIG. 1 with the retainer and anchor secured to tissue.

### DETAILED DESCRIPTION

Aspects of the disclosure are described hereinbelow with reference to the accompanying drawings; however, it is to be understood that the disclosed aspects are merely exemplary of the disclosure and may be embodied in various forms. Well-known functions or constructions are not described in detail to avoid obscuring the disclosure in unnecessary detail. Therefore, specific structural and functional details disclosed herein are not to be interpreted as limiting, but merely as a basis for the claims and as a representative basis for teaching one skilled in the art to variously employ the disclosure in virtually any appropriately detailed structure.

Descriptions of technical features of an aspect of the disclosure should typically be considered as available and applicable to other similar features of another aspect of the disclosure. Accordingly, technical features described herein according to one aspect of the disclosure may be applicable to other aspects of the disclosure, and thus duplicative descriptions may be omitted herein.

Aspects of the disclosure will be described more fully below (e.g., with reference to the accompanying drawings). Like reference numerals may refer to like elements throughout the specification and drawings.

Many access assemblies include an anchor mechanism for preventing withdrawal of the access assembly. These anchor mechanisms may be in the form of an inflatable balloon. Alternatively, the access assemblies may be maintained in position with an expandable flange or other structure capable of being collapsed to facilitate insertion of the access assembly through the tissue and selectively expanded to prevent withdrawal of the access assembly from the tissue.

The surgical access device of the present disclosure includes a retainer configured to engage tissue to help maintain the cannula in its position relative to the body during use.

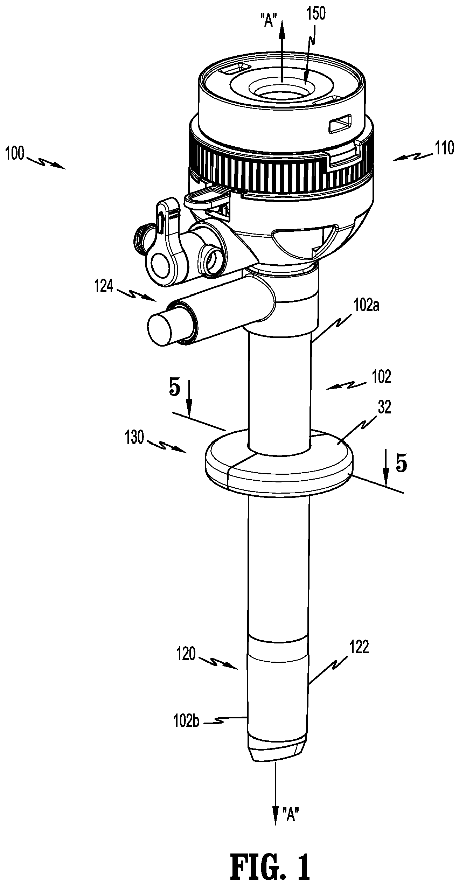

FIG. 1 generally illustrates a surgical access device 100 suitable for use with a retention mechanism according to an aspect of the present disclosure. The surgical access device 100 includes a cannula 102 and an instrument valve housing 110 supported by a proximal portion 102a of the cannula 102. Although shown including the instrument valve housing 110, it is envisioned that retention mechanisms in accordance with the disclosure may be incorporated into access assemblies without the instrument valve housing 110.

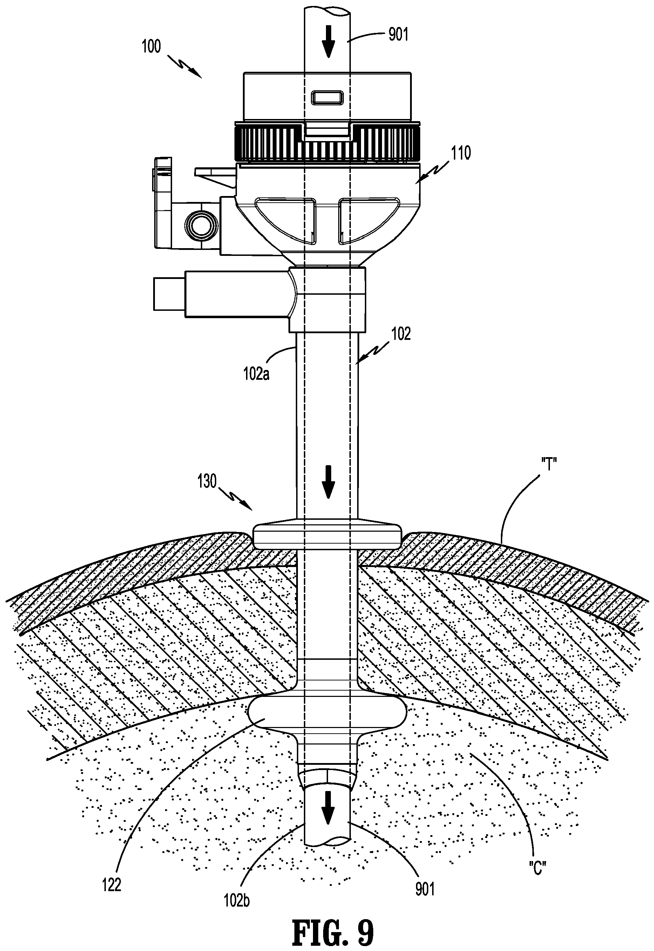

An obturator (not shown) is insertable through a channel 150 extending through the cannula 102 along longitudinal axis "A-A" to form or enlarge a surgical opening. A surgical instrument 901 (FIG. 9) may be inserted through the channel 150 after removal of the obturator. The surgical instrument 901 may be, for example, forceps, scissors, probes, dissectors, hooks, or retractors.

The surgical access device 100 includes an anchor 120 supported on a distal portion 102b of the cannula 102. As shown, the anchor 120 includes a balloon anchor 122. The balloon anchor 122 includes an uninflated or collapsed condition (FIG. 1) and an inflated or expanded condition (FIG. 9). The balloon anchor 122 is inflatable through a port 124 supported on the proximal portion 102a of the cannula 102. The port 124 fluidly couples the balloon anchor 122 with a source of inflation fluid (not shown). The anchor 120 will only be described herein to the extent necessary to disclose the features of the retention mechanisms of the present disclosure. For detailed descriptions of the structure and function of exemplary anchor assemblies suitable for use with the access assemblies the present disclosure, please refer to U.S. Pat. Nos. 5,697,946, 7,691,089, and 10,327,809, and U.S. Pat. App. Pub. No. 2004/0138702. Although features of the retention mechanisms of the present disclosure will be shown and described with reference to a balloon anchor, it is envisioned that the retention mechanisms of the present of the disclosure may be used with access assemblies having various anchor mechanism, including, for example, a collapsible flange.

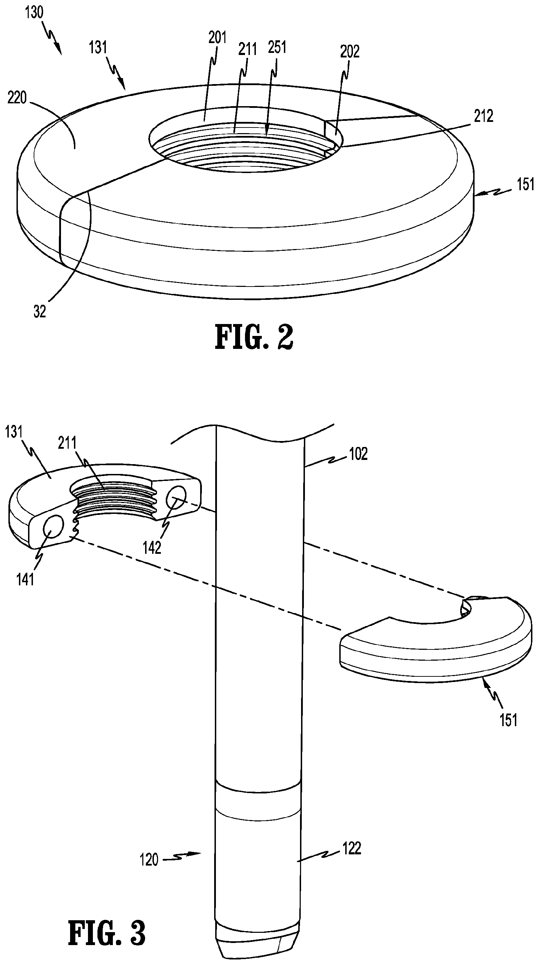

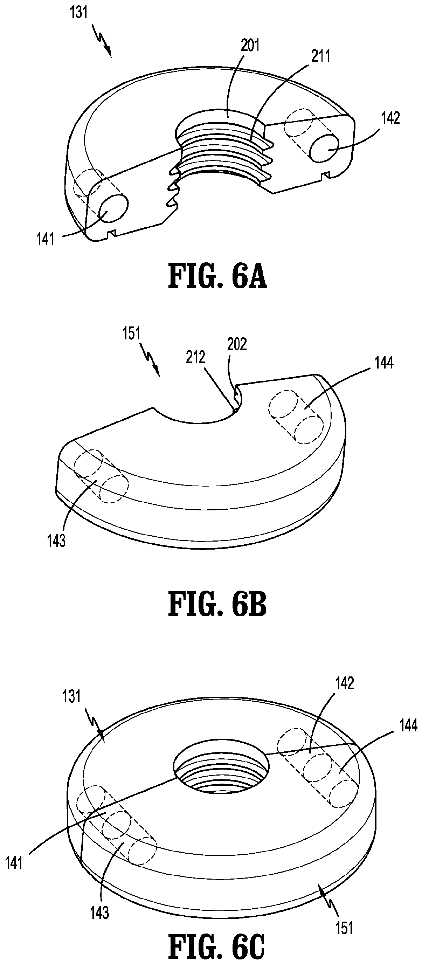

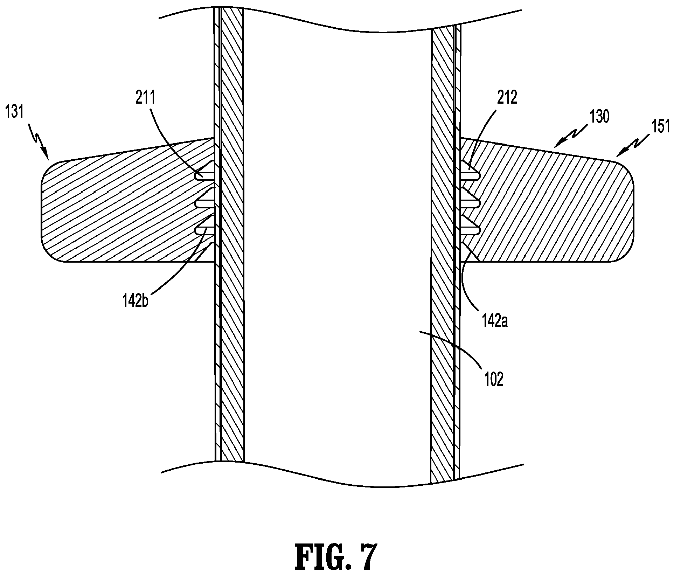

FIGS. 2-7 illustrate a retainer 130 according to an aspect of the present disclosure. The retainer 130 includes an annular body 32 configured to frictionally engage the cannula 102 of the surgical access device 100 to prevent over-insertion of the surgical access device 100 through tissue (see, e.g., tissue "T" in FIG. 9) during receipt and or manipulation of a surgical instrument (see, e.g., surgical instrument 901 in FIG. 9) through the surgical access device 100.

The retainer 130 includes a first member 131 and a second member 151. Each of the first and second members 131, 151 defines a semi-circular shape. The first member 131 includes a first arm 132 having a first lateral surface 133, and a second arm 134 having a second lateral surface 135. A first magnet 141 is positioned in the first arm 132 and a second magnet 142 is positioned in the second arm 134. The second member 151 defines a third arm 152 having a third lateral surface 153, and a fourth arm 154 having a fourth lateral surface 155. A third magnet 143 is positioned in the third arm 152 and a fourth magnet 144 is positioned in the fourth arm 154.

The first lateral surface 133 of the first member 131 is configured to contact the third lateral surface 153 of the second member 151. The second lateral surface 135 of the first member 131 is configured to contact the fourth lateral surface 155 of the second member 151. A first attractive force between the first magnet 141 and the third magnet 143, and a second attractive force between the second magnet 142 and the fourth magnet 144 releaseably secures the first member 131 to the second member 151 to removably frictionally engage the retainer 130 with the cannula 102. The first attractive force between the first magnet 141 and the third magnet 143 is generated by orienting the first magnet 141 such that a side of the first magnet 141 facing the third magnet 143 has an opposite polarity from a side of the third magnet 143 facing the first magnet 141. The second and fourth magnets 142, 144 are similarly arranged. This arrangement allows the first and second members 131 and 151 to be held together by the magnets 141-144. Magnets are selected to have sufficient attractive force to hold the first and second members 131, 151 together, but also to allow separation of the first and second members 131, 151 by a force applied from a user's hands. Additionally, the magnets are selected to have sufficient attractive force to restrain access assembly 100 from longitudinal movement. As an example, neodymium magnets may be employed.

The lateral surfaces 133, 135, 153 and 155 may each extend substantially perpendicularly to an upper surface 220 of the retainer 130.

When the first member 131 and the second member 151 are coupled together, they define an opening 251 configured to receive the cannula 102 in a friction fit manner. The first member 131 and the second member 151 may be substantially similar in shape and dimensions, as shown, to reduce the manufacturing cost and facilitate assembly of the retainer 130.

The first member 131 includes a first inner surface 201 defining first ridges 211. The second member 151 includes a second inner surface 202 defining second ridges 212. The first and second ridges 211, 212 are configured to frictionally engage the cannula 102 by gripping an outer surface of the cannula 102 to limit movement of the retainer 130 relative to the cannula 102.

The first and second ridges 211, 212 may permit longitudinal movement of the retainer 130 in a distal direction, e.g., towards the anchor 120. For example, the first and second ridges 211, 212 include angled distal facing surfaces 142a and flat proximal facing surfaces 142b. The angled surfaces 142a accommodate flexing of the ridges 211, 212 when a distal force is applied to the retainer 130 to permit distal movement of the retainer 130 relative to the cannula 102, and the flat surfaces 142b inhibit flexing of the ridges 211, 212 when a proximal force is applied to the retainer 130 to inhibit or limit proximal movement of the retainer 130 relative to the cannula 102. In this manner, once the retainer 130 is secured to the cannula 102, the retainer 130 may be moved distally to sandwich the tissue, e.g., of the abdominal wall, between the anchor 120 (FIG. 9) and the retainer 130 to ensure the surgical access assembly 100 remains fixed relative to the tissue throughout a surgical procedure.

The retainer 130 may be configured for releasable engagement of the cannula 102 of the surgical access assembly 100. For example, the first and second members 131, 151 may include finger grips or openings (not shown) to facilitate engagement by a user to permit pulling the first and second members 131, 151 apart. Alternatively, the retainer 130 may remain secured to the access assembly 100 and be disposed of along with the cannula 102 following a surgical procedure.

The retainer 130 may include or be formed of rubber, plastic, or a polymer.

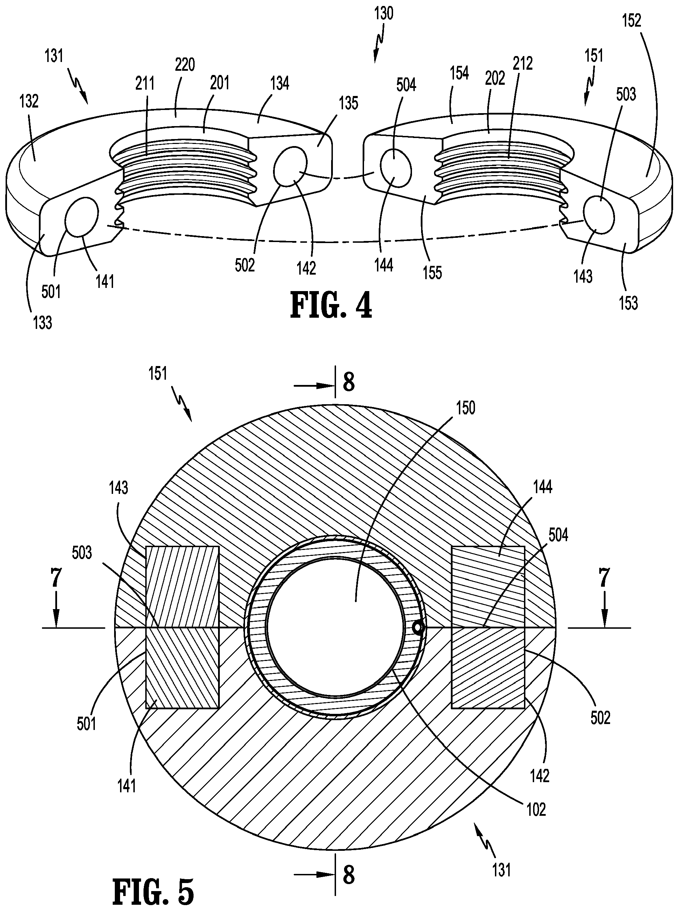

Referring particularly to FIGS. 4 and 5, a first outer surface 501 of the first magnet 141 defines a first continuously planar surface with the first lateral surface 133 of the first member 131. A second outer surface 502 of the second magnet 142 forms a second continuously planar surface with the second lateral surface 135 of the first member 131. A third outer surface 503 of the third magnet 143 forms a third continuously planar surface with the third lateral surface 153 of the second member 151. A fourth outer surface 504 of the fourth magnet 144 forms a fourth continuously planar surface with the fourth lateral surface 155 of the second member 151 (e.g., along cross-sectional line 7-7 of FIG. 5). Alternatively, the magnets 141-144 may each be recessed with respect to corresponding lateral surfaces 133, 135, 153 or 155 inside the respective first or second members 131, 151 such that the opposite polarities of the magnets 141-144 still create the attractive force between magnets 141 and 143 and between magnets 142 and 144. Each of these arrangements allow the first and second members 131 and 151 to be held together by the magnets 141-144. Magnets are selected to have sufficient attractive force to hold the first and second members 131, 151 together, but also to allow separation of the first and second members 131, 151 by a force applied from a user's hands. Additionally, the magnets are selected to have sufficient attractive force to restrain access assembly 100 from longitudinal movement. As an example, neodymium magnets may be employed.

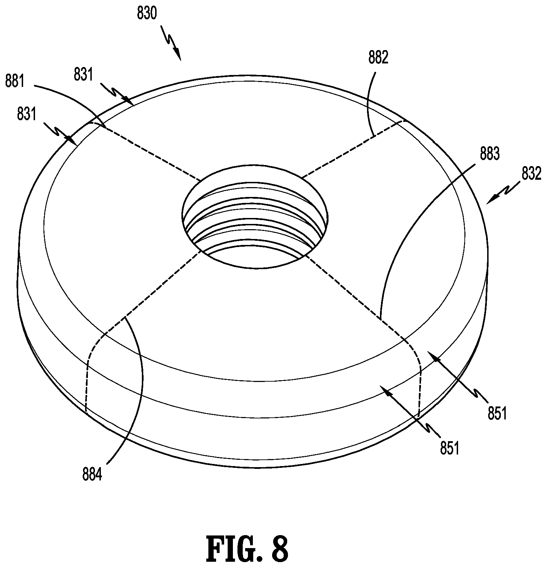

Referring to FIG. 8, according to another aspect of the present disclosure, the surgical access device 100 includes a retainer 830 configured to be removably frictionally engaged with the cannula 102. The retainer 830 includes an annular body 832 defining a first body portion 831 and a second body portion 851. A serrated edge (e.g., including serrated edges 882, 884) is formed in the annular body 832 between the first body portion 831 and the second body portion 851. When a serrated edge extending along a single line is employed (e.g., including serrated edges 882, 884), the annular body 832 is divided into two approximately equal sized sections, each occupying about 180° of circumference of the annular body 832. Additional serrated edges (e.g., serrated edge 881 and/or 883) may also be formed in the annular body 832. In an example in which four serrated edges are employed, the annular body 832 may be divided into four approximately equal sized sections, each occupying about 90° of circumference of the annular body 832.

Each serrated edge is configured to facilitate cutting of the annular body 832 (e.g., by a surgical tool such as scissors or a scalpel) into multiple sections (e.g., into two sections or four sections). For example, the serrated edges 882, 884 may be cut to separate the first body portion 831 from the second body portion 851 to remove the retainer 830 from the cannula 102.

Each serrated edge may also be configured to "snap apart" the sections of the annular body 832. For example, the first body portion 831 may be separated from the second body portion 851 by applying bending pressure along the serrated edge 882, 884 to create a linear break along serrated edges 882, 884.

FIG. 9 illustrates the surgical access assembly 100 received through and secured to tissue "T", e.g., an abdominal wall. More particularly, after the distal portion 102b of the cannula 102 of the surgical access assembly 100 is received through tissue "T", using an obturator (not shown) or other method, the balloon anchor 122 of the anchor 120 is inflated within a cavity "C" to prevent the cannula 102 from being withdrawn through the tissue "T". The retainer 130 may be secured to the cannula 102 subsequent to distal portion 102b the cannula 102 being received through the tissue "T" or prior to the distal portion 102b of the cannula being received through tissue "T". The retainer 130 may be provided to the surgeon already secured to the cannula 102 of the surgical access assembly 100 or may be secured to the cannula 102 in the operating room prior to receipt of the cannula through the tissue "T".

The retainer 130 is secured to the cannula 102 of the access assembly 100 by joining the first member 131 and the second member 151 about the cannula 102 to connect the corresponding first and third magnets 141, 143 and the corresponding second and fourth magnets 142, 144.

Following inflation of the balloon anchor 122 of the anchor 120, the retainer 130 may be slid distally along the cannula 102 to sandwich the tissue "T" between the anchor 120 and the retainer 130. In this manner, the surgical access assembly 100 is secured to the tissue "T" and the access assembly 100 is inhibited from longitudinal movement relative to the tissue "T" throughout insertion, withdrawal, and/or manipulation of a surgical instrument "I" through the surgical access assembly 100.

Following a surgical procedure, the balloon anchor 122 of the anchor 120 may be deflated to permit withdrawal of the cannula 102 of the surgical access assembly 100 from the tissue "T". As noted above, the retainer 130 may be configured to be removed from the cannula 102 prior to or subsequent to removal of the access assembly from the tissue "T". Alternatively, the retainer 130 may remain secured to and disposed with the cannula 102 of the surgical access assembly 100.

While the above description contains many specifics, these specifics should not be construed as limitations on the scope of the present disclosure, but merely as illustrations of various aspects thereof. Therefore, the above description should not be construed as limiting, but merely as exemplifications of various aspects. Those skilled in the art will envision other modifications within the scope and spirit of the claims.

THE INVENTION IS DESCRIBED BY THE FOLLOWING NUMBERED PARAGRAPHS:-
1. A surgical access device, comprising:
   an instrument valve housing including a cannula extending distally from the instrument valve housing, the cannula defining a longitudinal axis;
   an anchor supported at a distal end portion of the cannula; and
   a retainer configured to be removably engaged with the cannula, the retainer including:
      a first member defining a semi-circular shape, the first member including a first arm having a first lateral surface and a second arm having a second lateral surface, wherein a first magnet is positioned in the first arm and a second magnet is positioned in the second arm; and
      a second member defining a semi-circular shape, the second member including a third arm having a third lateral surface and a fourth arm having a fourth lateral surface, wherein a third magnet is positioned in the third arm and a fourth magnet is positioned in the fourth arm,
      wherein the first lateral surface of the first member is configured to contact the third lateral surface of the second member, and the second lateral surface of the first member is configured to contact the fourth lateral surface of the second member, and
      wherein a first attractive force between the first magnet and the third magnet, and a second attractive force between the second magnet and the fourth magnet releaseably couples the first member to the second member such that the retainer frictionally engages with the cannula.
2. The surgical access device of paragraph 1, wherein the first member includes a first inner surface defining first ridges and the second member includes a second inner surface defining second ridges, wherein the first and second ridges are configured to frictionally engage the cannula.
3. The surgical access device of paragraph 1, wherein a first outer surface of the first magnet defines a first continuously planar surface with the first lateral surface of the first member, a second outer surface of the second magnet forms a second continuously planar surface with the second lateral surface of the first member, a third outer surface of the third magnet forms a third continuously planar surface with the third lateral surface of the second member, and a fourth outer surface of the fourth magnet forms a fourth continuously planar surface with the fourth lateral surface of the second member.
4. The surgical access device of paragraph 1, wherein the anchor includes a balloon anchor.
5. The surgical access device of paragraph 4, further including a port supported on the instrument valve housing, the port configured to provide inflation fluid to the balloon anchor.
6. The surgical access device of paragraph 1, wherein the retainer is formed of rubber, plastic, or a polymer.
7. A retainer for use with a cannula, comprising:
   a first member having a semi-circular shape, the first member including a first arm having a first lateral surface and a second arm having a second lateral surface, wherein a first magnet is positioned in the first arm and a second magnet is positioned in the second arm; and
   a second member having a semi-circular shape, the second member including a third arm having a third lateral surface and a fourth arm having a fourth lateral surface, wherein a third magnet is positioned in the third arm and a fourth magnet is positioned in the fourth arm,
   wherein the first lateral surface of the first member is configured to contact the third lateral surface of the second member, and the second lateral surface of the first member is configured to contact the fourth lateral surface of the second member, and
   wherein a first attractive force between the first magnet and the third magnet, and a second attractive force between the second magnet and the fourth magnet removably couples the first member to the second member to frictionally engage the retainer to the cannula.
8. The retainer of paragraph 7, wherein the first member includes a first inner surface defining first ridges and the second member includes a second inner surface defining second ridges, wherein the first and second ridges are configured to frictionally engage the cannula.
9. The retainer of paragraph 7, wherein a first outer surface of the first magnet defines a first continuously planar surface with the first lateral surface of the first member, a second outer surface of the second magnet forms a second continuously planar surface with the second lateral surface of the first member, a third outer surface of the third magnet forms a third continuously planar surface with the third lateral surface of the second member, and a fourth outer surface of the fourth magnet forms a fourth continuously planar surface with the fourth lateral surface of the second member.
10. The retainer of paragraph 7, wherein the retainer is formed of rubber, plastic, or a polymer.
11. A surgical access device, comprising:
   an instrument valve housing including a cannula extending distally from the instrument valve housing, the cannula defining a longitudinal axis;
   an anchor supported at a distal end portion of the cannula; and
   a retainer configured to be frictionally engaged with cannula, the retainer including an annular body having a first body portion and a second body portion,
   wherein a serrated edge is formed in the annular body between the first body portion and the second body portion, the serrated edge configured to facilitate cutting of the annular body to separate the first body portion from the second body portion to remove the retainer from the cannula.
12. The surgical access device of paragraph 11, further including a second serrated edge formed in the first body portion and a third serrated edge formed in the second body portion.
13. The surgical access device of paragraph 11, wherein the retainer is formed of rubber, plastic, or a polymer.

## Claims

1. A surgical access device, comprising:
an instrument valve housing including a cannula extending distally from the instrument valve housing, the cannula defining a longitudinal axis;
an anchor supported at a distal end portion of the cannula; and
a retainer configured to be removably engaged with the cannula, the retainer including:
a first member defining a semi-circular shape, the first member including a first arm having a first lateral surface and a second arm having a second lateral surface, wherein a first magnet is positioned in the first arm and a second magnet is positioned in the second arm; and
a second member defining a semi-circular shape, the second member including a third arm having a third lateral surface and a fourth arm having a fourth lateral surface, wherein a third magnet is positioned in the third arm and a fourth magnet is positioned in the fourth arm,
wherein the first lateral surface of the first member is configured to contact the third lateral surface of the second member, and the second lateral surface of the first member is configured to contact the fourth lateral surface of the second member, and
wherein a first attractive force between the first magnet and the third magnet, and a second attractive force between the second magnet and the fourth magnet releaseably couples the first member to the second member such that the retainer frictionally engages with the cannula.

2. The surgical access device of claim 1, wherein the first member includes a first inner surface defining first ridges and the second member includes a second inner surface defining second ridges, wherein the first and second ridges are configured to frictionally engage the cannula.

3. The surgical access device of claim 1 or claim 2, wherein a first outer surface of the first magnet defines a first continuously planar surface with the first lateral surface of the first member, a second outer surface of the second magnet forms a second continuously planar surface with the second lateral surface of the first member, a third outer surface of the third magnet forms a third continuously planar surface with the third lateral surface of the second member, and a fourth outer surface of the fourth magnet forms a fourth continuously planar surface with the fourth lateral surface of the second member.

4. The surgical access device of any preceding claim, wherein the anchor includes a balloon anchor.

5. The surgical access device of claim 4, further including a port supported on the instrument valve housing, the port configured to provide inflation fluid to the balloon anchor.

6. The surgical access device of any preceding claim, wherein the retainer is formed of rubber, plastic, or a polymer.

7. A retainer for use with a cannula, comprising:
a first member having a semi-circular shape, the first member including a first arm having a first lateral surface and a second arm having a second lateral surface, wherein a first magnet is positioned in the first arm and a second magnet is positioned in the second arm; and
a second member having a semi-circular shape, the second member including a third arm having a third lateral surface and a fourth arm having a fourth lateral surface, wherein a third magnet is positioned in the third arm and a fourth magnet is positioned in the fourth arm,
wherein the first lateral surface of the first member is configured to contact the third lateral surface of the second member, and the second lateral surface of the first member is configured to contact the fourth lateral surface of the second member, and
wherein a first attractive force between the first magnet and the third magnet, and a second attractive force between the second magnet and the fourth magnet removably couples the first member to the second member to frictionally engage the retainer to the cannula.

8. The retainer of claim 7, wherein the first member includes a first inner surface defining first ridges and the second member includes a second inner surface defining second ridges, wherein the first and second ridges are configured to frictionally engage the cannula.

9. The retainer of claim 7 or claim 8, wherein a first outer surface of the first magnet defines a first continuously planar surface with the first lateral surface of the first member, a second outer surface of the second magnet forms a second continuously planar surface with the second lateral surface of the first member, a third outer surface of the third magnet forms a third continuously planar surface with the third lateral surface of the second member, and a fourth outer surface of the fourth magnet forms a fourth continuously planar surface with the fourth lateral surface of the second member.

10. The retainer of any of claims 7 to 9, wherein the retainer is formed of rubber, plastic, or a polymer.

11. A surgical access device, comprising:
an instrument valve housing including a cannula extending distally from the instrument valve housing, the cannula defining a longitudinal axis;
an anchor supported at a distal end portion of the cannula; and
a retainer configured to be frictionally engaged with cannula, the retainer including an annular body having a first body portion and a second body portion,
wherein a serrated edge is formed in the annular body between the first body portion and the second body portion, the serrated edge configured to facilitate cutting of the annular body to separate the first body portion from the second body portion to remove the retainer from the cannula.

12. The surgical access device of claim 11, further including a second serrated edge formed in the first body portion and a third serrated edge formed in the second body portion.

13. The surgical access device of claim 11 or claim 12, wherein the retainer is formed of rubber, plastic, or a polymer.
